# FASCICULE DE BREVET EUROPEEN

(11) **EP 1 623 729 B1**
(45) Date de publication et mention de la délivrance du brevet: **18.02.2009**
(21) Numéro de dépôt: 05291584.0
(22) Date de dépôt: 25.07.2005
(51) Int. Cl.: A61M 1/02, G05D 11/13

(54) **Machine de mélange en continue et selon un ratio déterminé d'un fluide biologique et d'une solution**
Gerät zum kontinuierlichen Mischen eines biologischen Fluids und einer Lösung in einem vorbestimmten Verhältnis
Apparatus for continuous mixing of a biologic fluid and a solution in a predetermined ratio

(30) Priorité: 03.08.2004 FR 0451769
(43) Date de publication de la demande: 08.02.2006
(73) Titulaire: MACO PHARMA, 59420 Mouvaux (FR)
(72) Inventeur: Behague, Maurice, 59126 Linselles (FR)
(74) Mandataire: Breese, Pierre

(56) Documents cités:
- EP-A- 0 583 148
- EP-A- 1 442 758
- EP-A- 1 442 759
- US-A- 4 582 598

## Description

L'invention concerne une machine de mélange en continu et selon un ratio déterminé d'un fluide biologique et d'une solution anticoagulante et/ou de conservation.

Elle s'applique typiquement au cas où le fluide biologique est du sang total qui doit être prélevé d'un donneur dans une poche de recueil. En effet, il est recommandé que le sang soit prélevé de façon stérile et soit mélangé avec une solution anticoagulante et/ou de conservation lors du prélèvement de sorte à permettre son utilisation ultérieure dans les meilleures conditions de sécurité sanitaire.

Un problème qui se pose concerne l'obtention d'un ratio déterminé entre la quantité de fluide prélevé et la quantité de solution anticoagulante et/ou de conservation. En effet, notamment dans le domaine du prélèvement sanguin, la quantité de solution anticoagulante et/ou de conservation présente dans la poche de recueil est fixée à une certaine valeur pour que le sang soit utilisable dans le domaine médical.

On connaît du document EP-A-0 583 148 un appareil d'introduction d'un flux de fluide médical dans un flux de fluide biologique qui comprend un moyen de commande conçu pour recevoir un signal de volume, pour calculer une vitesse de changement de volume et pour régler le débit de fluide médical en fonction du changement de vitesse calculé.

Ce type de stratégie pour obtenir un ratio déterminé de fluide médical dans un fluide biologique ne donne pas entièrement satisfaction. En effet, le réglage du débit de fluide médical est basé sur la mesure d'une différence de volume qui ne peut pas être réalisée de façon satisfaisante du fait de la trop faible résolution des instruments de mesure utilisables.

Ainsi, l'opérateur a deux choix possibles pour mettre en oeuvre ce type de machine :
- utiliser une période de mesure importante pour pouvoir obtenir de façon relativement précise la variation de volume, mais alors le réglage du débit de fluide médical n'est pas réalisé de façon assez fréquente pour pouvoir s'accorder de façon satisfaisante au débit de fluide biologique. En particulier, en cas de variation importante dans le débit de fluide biologique, le réglage du débit de fluide médical est trop brutal, de sorte que le ratio ne peut pas être conservé au cours du temps de façon satisfaisante ;
- utiliser une période de mesure plus faible pour tenter de mieux conserver le ratio au cours du temps, mais alors la précision de mesure de la variation de volume est trop faible pour obtenir un réglage satisfaisant du débit de fluide médical. En particulier, l'opérateur peut alors observer des instabilités dans le réglage, ce qui sollicite de façon importante la machine et, in fine, ne permet pas de conserver le ratio au cours du temps.

L'invention vise à résoudre ces problèmes en proposant notamment une machine de mélange en continu et selon un ratio déterminé d'un fluide biologique et d'une solution anticoagulante et/ou de conservation, qui permet une meilleure conservation du ratio au cours du mélange, et ce en utilisant des instruments de mesure de résolution standard.

A cet effet l'invention concerne une machine de mélange en continu et selon un ratio déterminé d'un fluide biologique, notamment sanguin, et d'une solution anticoagulante et/ou de conservation, ladite machine comprenant un dispositif de mesure du volume de fluide qui est prélevé par écoulement naturel, un dispositif de pompage de la solution anticoagulante et/ou de conservation et un système de pilotage du dispositif de pompage comprenant un dispositif de détermination du volume de solution pompée et un dispositif d'asservissement de la vitesse de pompage qui comprend des moyens aptes à :
- en fonction du ratio déterminé, calculer le volume théorique de solution à mélanger au volume de fluide prélevé ;
- comparer le volume théorique de solution et le volume de solution pompée;
- ajuster la vitesse de pompage en fonction de la comparaison précédente de sorte à tendre vers le ratio déterminé entre la quantité de fluide prélevé et la quantité de solution anticoagulante et/ou de conservation mélangée.

D'autres objets et avantages de l'invention apparaîtront au cours de la description qui suit en référence aux dessins annexés.

La figure 1 représente de façon schématique le fonctionnement d'une machine de mélange selon une première réalisation dans laquelle seule la poche de recueil est disposée sur un moyen de mesure de poids.

La figure 2 représente de façon schématique le fonctionnement d'une machine de mélange selon une autre réalisation dans laquelle les poches de recueil et de solution anticoagulante et/ou de conservation sont disposées sur un moyen de mesure de poids.

La figure 3 représente de façon schématique une vue en perspective de la machine de mélange selon une réalisation.

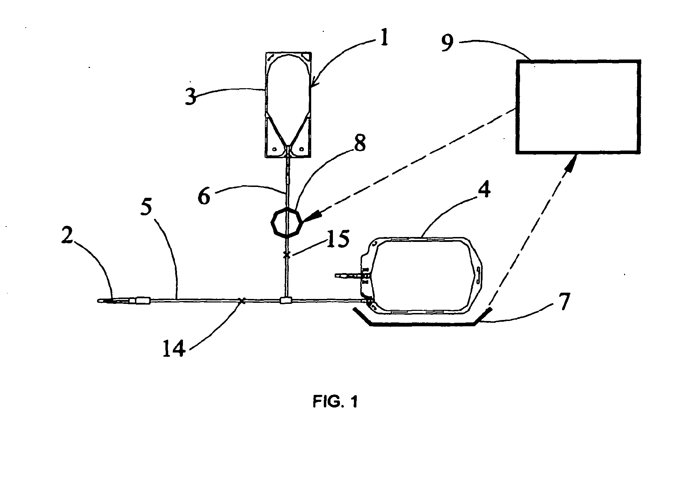

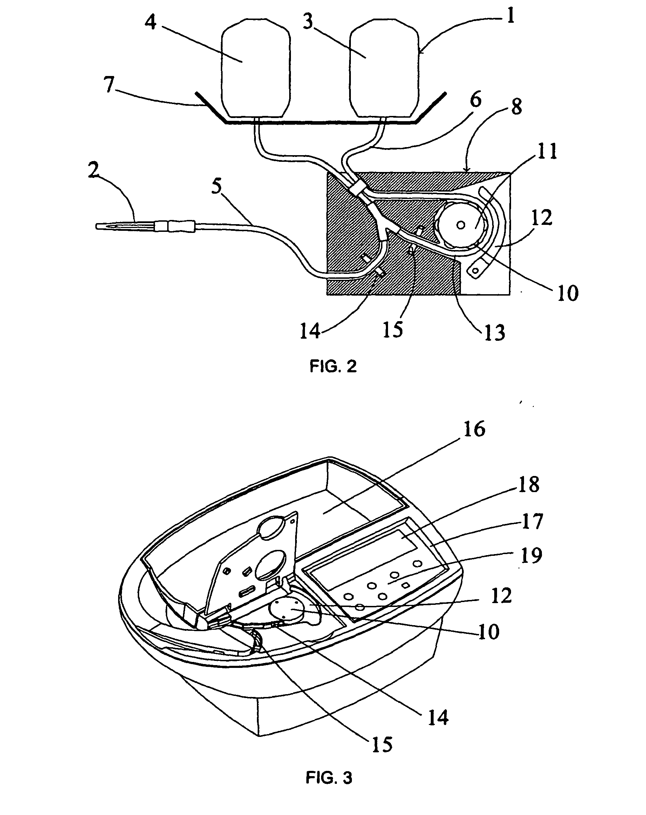

Les figures 1 et 2 représentent le principe de fonctionnement d'une machine de mélange en continu et selon un ratio déterminé d'un fluide biologique et d'une solution anticoagulante et/ou de conservation.

Notamment, le fluide biologique est un fluide sanguin tel que du sang total prélevé d'un donneur et la solution anticoagulante et/ou de conservation est une solution de type CPD (citrate phosphate dextrose), CP2D (citrate phosphate 2 dextrose) ou ACD (acide citrate dextrose). Généralement, le ratio déterminé entre la quantité de solution anticoagulante et/ou de conservation et la quantité de sang total est fixé à 1/7. Toutefois, ce ratio varie selon les zones géographiques dans lesquelles le sang est prélevé.

Selon la figure 1 et 2, le fluide est prélevé d'un donneur par écoulement naturel. Le mélange du fluide et de la solution anticoagulante et/ou de conservation est réalisé à l'aide d'un système à poches 1 comprenant des moyens de prélèvement 2 du fluide, une poche 3 contenant une solution anticoagulante et/ou de conservation du fluide prélevé, et une poche de recueil 4 destinée à recevoir le fluide prélevé mélangé avec la solution anticoagulante et/ou de conservation. La poche de recueil 4 est en communication fluidique avec les moyens de prélèvement 2 par l'intermédiaire d'une première tubulure 5 souple et avec la poche 3 contenant la solution anticoagulante et/ou de conservation par l'intermédiaire d'une deuxième tubulure 6 souple.

Les moyens de prélèvement 2 sont constitués notamment d'une aiguille permettant l'accès à la veine du donneur. Dans ce cas, le fluide est prélevé directement du donneur. Le prélèvement est réalisé par écoulement naturel, c'est-à-dire basé sur la pression veineuse et/ou la gravité. Ce mode de prélèvement offre un confort et une sécurité supérieurs au donneur.

Sur les figures 1 et 2, tous les éléments du système à poches 1 sont préconnectés de sorte à former un système unitaire fermé ou en circuit clos. Le système ainsi formé est stérilisé, conditionné dans un emballage stérile et prêt-à-l'emploi.

En variante non représentée, le fluide biologique destiné à être mélangé est contenu dans une poche de transfert. Dans ce cas, les moyens de prélèvement 2 comprennent un perforateur pouvant se connecter à un orifice de sortie de la poche de transfert contenant le fluide biologique. Le fluide biologique est prélevé de la poche de transfert par écoulement nature, par exemple par gravité.

Selon les figures 1 et 2, la machine de mélange comprend un dispositif de mesure 7 du volume de fluide qui est prélevé par écoulement naturel, un dispositif de pompage 8 de la solution anticoagulante et/ou de conservation et un système de pilotage 9 du dispositif de pompage.

Le dispositif de mesure 7 permet de connaître à n'importe quel moment le volume total de fluide prélevé par écoulement naturel.

Selon la réalisation représentée sur la figure 1, le dispositif de mesure 7 du volume de fluide prélevé comprend un moyen de mesure du poids de fluide prélevé telle qu'une balance et un moyen de calcul du volume correspondant. Le passage du poids de fluide au volume de fluide se calcule à l'aide de la densité du fluide.

Selon la figure 2, l'ensemble du système à poches 1 est disposé sur la balance. Une tare est effectuée par la balance après le placement du système à poches 1, afin de tenir compte du poids dudit système à poches avant le mélange. L'anticoagulant et/ou la solution de conservation passant ensuite de la poche 3 de solution anticoagulante et/ou de conservation à la poche de recueil 4, ces deux poches étant sur la balance, seul le poids du fluide prélevé est mesuré.

Si seule la poche de recueil 4 est placée sur la balance (figure 1), il faut tenir compte en plus de la tare normalement effectuée, d'une constante qui est la variation de poids liée à l'introduction de la solution anticoagulante et/ou de conservation dans la poche de recueil 4.

Selon la figure 2, le dispositif de pompage comprend une pompe péristaltique 10 à une seule tête 11 qui est mobile en rotation à vitesses variables.

La machine de mélange comprend alors un dispositif de placement 12, autour d'une partie de la tête 11, d'une boucle d'écoulement 13 de la solution de sorte à permettre la circulation de la solution par écrasement partiel d'une zone de ladite boucle.

La boucle d'écoulement est formée par une partie de la deuxième tubulure 6 reliant la poche 3 de solution anticoagulante et/ou de conservation à la poche de recueil 4.

Cette boucle peut être préformée comme dans le système à poches décrit dans la demande de brevet européen EP-A-1 442 759.

Selon l'invention, le système de pilotage 9 comprend un dispositif de détermination du volume de solution pompée et un dispositif d'asservissement de la vitesse de pompage.

Le dispositif de détermination du volume permet de connaître à n'importe quel moment depuis le début du mélange le volume total de solution anticoagulante et/ou de conservation pompée.

Selon la figure 2, le dispositif de détermination du volume de solution pompée comprend un moyen de détermination du nombre de tours effectués par la tête 11 de le pompe péristaltique 10, et un moyen de calcul du volume pompé correspondant audit nombre de tours.

Le dispositif de mesure du volume de fluide prélevé ou le dispositif de détermination du volume de solution pompée détermine n'importe quel paramètre du fluide ou de la solution à partir duquel un volume peut être calculé à l'aide d'algorithme. Par exemple, le dispositif de mesure du volume ou de détermination du volume détectent une masse, un débit, une hauteur dans une poche, une pression et/ou une déformation.

Ainsi, le dispositif peut comprendre des détecteurs optiques, des capteurs de débit, des capteurs de pression...

Notamment, le dispositif de mesure du volume du fluide prélevé ou le dispositif de détermination du volume de la solution pompée préserve la stérilité du système à poches.

Le système de pilotage 9 selon l'invention comprend en outre un dispositif d'asservissement de la vitesse de pompage, par exemple sous la forme d'un calculateur, qui comprend des moyens aptes à :
- en fonction du ratio déterminé, calculer le volume théorique de solution à mélanger au volume de fluide prélevé ;
- comparer le volume théorique de solution et le volume de solution pompée ;
- ajuster la vitesse de pompage en fonction de la comparaison précédente de sorte à tendre vers le ratio déterminé entre la quantité de fluide prélevé et la quantité de solution anticoagulante et/ou de conservation mélangée.

Le dispositif d'asservissement de la vitesse de pompage permet d'asservir la vitesse du dispositif de pompage en fonction du volume théorique de solution et du volume de solution pompée. Par exemple, le dispositif d'asservissement contrôle le moteur de la pompe 10.

Des capteurs optiques 14,15 sont ajoutés à la machine. Un premier capteur optique 14 est placé sur la première tubulure 5. Ce capteur permet de détecter la présence de fluide, afin de vérifier que le fluide circule convenablement au sein de la première tubulure 5. Il permet également de vérifier s'il n'y a pas d'air ou de solution anticoagulante et/ou de conservation remontant vers les moyens de prélèvement. Ce capteur optique 14 peut par exemple être remplacé ou complété par un capteur à ultrasons permettant de détecter de façon plus fine les inversions de flux.

Un deuxième capteur optique 15 peut être placé sur la deuxième tubulure 6. Ce capteur permet de détecter la présence de solution anticoagulante et/ou de conservation, afin de vérifier que la solution anticoagulante et/ou de conservation circule convenablement au sein de la deuxième tubulure 6.

La figure 3 représente une vue en perspective d'une machine de mélange de sang et de solution anticoagulante et/ou de conservation. La machine de mélange comprend un moyen de mesure du poids de fluide prélevé sous forme de balance 16, une pompe péristaltique 10 et un système de pilotage de la pompe péristaltique tel que décrit ci-dessus.

La machine comprend en outre une interface 17 pour l'utilisateur comprenant un afficheur 18 et un clavier 19.

La machine comprend également un dispositif de placement 12, autour d'une partie de la tête 11, d'une boucle d'écoulement de la solution de sorte à permettre la circulation de la solution par écrasement partiel d'une zone de ladite boucle.

Le procédé pour mélanger un fluide biologique et une solution anticoagulante prévoit d'utiliser un système à poches 1 tel que décrit en relation avec la figure 1 comprenant, en circuit clos, des moyens de prélèvement 2 du fluide, une poche 3 contenant une solution anticoagulante et/ou de conservation du fluide prélevé, et une poche de recueil 4 destinée à recevoir le fluide prélevé mélangé avec la solution anticoagulante et/ou de conservation. La poche de recueil 4 est en communication fluidique avec les moyens de prélèvement 2 par l'intermédiaire d'une première tubulure 5 souple et avec la poche 3 contenant la solution anticoagulante et/ou de conservation par l'intermédiaire d'une deuxième tubulure 6 souple.

Le procédé comprend les étapes itératives suivantes :
- mesurer le volume de fluide prélevé dans la poche de recueil 4;
- déterminer le volume de solution pompée depuis la poche 3 la contenant ;
- en fonction du ratio déterminé, calculer le volume théorique de solution à mélanger au volume de fluide prélevé ;
- comparer le volume théorique de solution et le volume de solution pompée;
- ajuster la vitesse de pompage en fonction de la comparaison précédente de sorte à tendre vers le ratio déterminé entre la quantité de fluide prélevé et la quantité de solution anticoagulante et/ou de conservation mélangée.

La mesure du volume de fluide prélevé est réalisée par disposition d'au moins la poche de recueil 4 sur un moyen de mesure du poids, puis calcul du volume de fluide correspondant au poids mesuré.

En relation avec la machine de mélange illustrée sur la figure 3, la poche de recueil et la poche de solution anticoagulante et/ou de conservation sont disposées sur la balance 16. La balance détermine ainsi directement le poids de fluide prélevé. La conversion du poids du fluide prélevé en volume de fluide prélevé est calculé à l'aide de la densité du sang.

Ce volume de fluide prélevé permet de calculer en fonction du ratio déterminé, un volume théorique de solution.

Parallèlement, le volume de solution pompée est déterminé par le dispositif de détermination, et notamment le nombre de tours réalisés par la tête de pompe 11 de la pompe péristaltique 10.

La différence entre le volume théorique de solution et le volume de solution pompée est calculée et l'ajustement de la vitesse de pompage est réalisé par application d'une vitesse de pompage qui est fonction de cette différence.

Par exemple, si la différence est négative, cela signifie que trop de solution a été pompée par rapport à la quantité de fluide prélevé, le pompage est alors interrompu.

Si la différence est positive, cela signifie que pas assez de solution a été pompée, le pompage a lieu. La vitesse de pompage est déterminée en fonction de cette différence. Plus la différence est grande, plus la vitesse est élevée pour rattraper de façon appropriée le manque de solution anticoagulante et/ou de conservation dans la poche de recueil par rapport au fluide prélevé.

En variante, le volume théorique de solution calculé à partir du volume de fluide prélevé est transcrit en nombre de tour théorique de la tête de la pompe péristaltique.

Le nombre de tours réellement réalisés par la tête de pompe est comparé à ce nombre de tour théorique de la tête de pompe et la vitesse de pompage est ajustée en fonction de la comparaison précédente.

Ces étapes itératives sont réalisées avec une période d'échantillonnage comprise entre 1 et 5 s, notamment 3 s.

Cette période d'échantillonnage est suffisamment longue pour permettre une variation du volume de fluide prélevé suffisante pour être détectée de façon précise et suffisamment courte pour obtenir quasi en continu le ratio déterminé entre la quantité de solution anticoagulante et/ou de conservation mélangée.

Ainsi, même si le mélange est interrompu pour quelque raison avant la fin de la procédure, le ratio déterminé est respecté. Dans le cas d'un prélèvement sanguin, la perte de ce ratio conduit à une perte de la qualité du sang par lyse des globules rouges et une détérioration de la fonctionnalité des plaquettes, d'où une impossibilité d'utiliser du sang.

La machine et le procédé associé sont particulièrement adaptés au prélèvement du sang d'un donneur dans lequel le volume de fluide prélevé augmente de façon croissante avec un débit limité, comme cela est le cas lors d'un prélèvement de sang total par écoulement naturel.

Le procédé décrit permet d'éviter les changements rapides de la vitesse de pompage.

## Revendications

1. Machine de mélange en continu et selon un ratio déterminé d'un fluide biologique, notamment sanguin, et d'une solution anticoagulante et/ou de conservation, ladite machine comprenant un dispositif de mesure (7) du volume de fluide qui est prélevé par écoulement naturel, un dispositif de pompage (8) de la solution anticoagulante et/ou de conservation et un système de pilotage (9) du dispositif de pompage, le système de pilotage comprenant un dispositif de détermination du volume de solution pompée et un dispositif d'asservissement de la vitesse de pompage **charactérisée en ce que** le dispositif d'asservissement de la vitesse de pompage comprend des moyens aptes à :
- en fonction du ratio déterminé, calculer le volume théorique de solution à mélanger au volume de fluide prélevé ;
- comparer le volume théorique de solution et le volume de solution pompée ;
- ajuster la vitesse de pompage en fonction de la comparaison précédente de sorte à tendre vers le ratio déterminé entre la quantité de fluide prélevé et la quantité de solution anticoagulante et/ou de conservation mélangée.

2. Machine de mélange selon la revendication 1, **caractérisée en ce que** le dispositif de mesure (7) du volume de fluide prélevé comprend un moyen de mesure du poids de fluide prélevé et un moyen de calcul du volume correspondant.

3. Machine de mélange selon la revendication 1 ou 2, **caractérisée en ce que** le dispositif de pompage (8) comprend une pompe péristaltique (10) à une seule tête (11) qui est mobile en rotation à vitesses variables.

4. Machine de mélange selon la revendication 3, **caractérisée en ce que** le dispositif de détermination du volume de solution pompée comprend un moyen de détermination du nombre de tours effectués par la tête (11), et un moyen de calcul du volume pompé correspondant audit nombre de tours.

5. Machine de mélange selon la revendication 3 ou 4, **caractérisée en ce qu'**elle comprend un dispositif de placement (12), autour d'une partie de la tête (11), d'une boucle d'écoulement (13) de la solution de sorte à permettre la circulation de la solution par écrasement partiel d'une zone de ladite boucle.

## Claims

1. A machine for continuously blending, according to a determined ratio of a biological fluid, particularly blood, and an anticoagulant and/or preserving solution, said machine comprising a device for measuring (7) the volume of the fluid sampled through a natural flow, a device for pumping (8) the anticoagulant and/or preserving solution and a system for driving (9) the pumping device, the driving system including a device for determining the volume of the pumped solution and a device for servo-controlling the pumping speed, **characterised in that** the device for servo-controlling the pumping speed includes means capable of:
- depending on the determined ratio, calculating the theoretical volume of the solution to be blended with the sampled volume of fluid;
- comparing the theoretical volume of the solution and the volume of the pumped solution;
- adjusting the pumping speed as a function of the preceding comparison, so as to tend towards the determined ratio between the quantity of the sampled fluid and the quantity of the blended anticoagulant and/or preserving solution.

2. A blending machine according to claim 1, **characterised in that** the device for measuring (7) the volume of the sampled fluid includes means for measuring the weight of the sampled fluid and means for calculating the corresponding volume.

3. A blending machine according to claim 1 or 2, **characterised in that** the pumping device (8) includes a peristaltic pump (10), with a single head (11), which is movable in rotation at variable speed.

4. A blending machine according to claim 3, **characterised in that** the device for determining the volume of the pump solution includes means for determining the number of revolutions made by the head (11), and means for calculating the pumped volume corresponding to said number of revolutions.

5. A blending machine according to claim 3 or 4, **characterised in that** it includes a device for positioning (12) a solution flow loop (13) about a part of the head (11), so as to enable the circulation of the solution through a partial crushing of one zone of said loop.

## Patentansprüche

1. Dauermischmaschine gemäß einem bestimmten Verhältnis einer biologischen Flüssigkeit, insbesondere Blut, und einer anti-koagulierenden Lösung und/oder Konservierungslösung, wobei die genannte Maschine eine Messvorrichtung (7) des durch natürliche Strömung entnommenen Flüssigkeitsvolumens, eine Pumpvorrichtung (8) der anti-koagulierenden Lösung und/oder Konservierungslösung und ein Steuersystem (9) der Pumpvorrichtung umfasst, wobei das Steuersystem eine Bestimmungsvorrichtung des gepumpten Lösungsvolumens und eine Regelvorrichtung der Pumpgeschwindigkeit umfasst, **dadurch gekennzeichnet, dass** die Regelvorrichtung der Pumpgeschwindigkeit Mittel umfasst, die:
- das mit dem entnommenen Flüssigkeitsvolumen zu mischende theoretische Lösungsvolumen gemäß dem bestimmten Verhältnis berechnen kann;
- das theoretische Lösungsvolumen und das gepumpte Lösungsvolumen vergleichen kann;
- die Pumpgeschwindigkeit entsprechend dem vorherigen Vergleich anpassen kann, um sie dem bestimmten Verhältnis zwischen der entnommenen Flüssigkeitsmenge und der Menge der gemischten anti-koagulierenden Lösung und/oder Konservierungslösung anzunähern.

2. Mischmaschine gemäß Anspruch 1, **dadurch gekennzeichnet, dass** die Messvorrichtung (7) des entnommenen Flüssigkeitsvolumens ein Messmittel des Gewichts der entnommenen Flüssigkeit und ein Berechnungsmittel des entsprechenden Volumens umfasst.

3. Mischmaschine gemäß Anspruch 1 oder 2, **dadurch gekennzeichnet, dass** die Pumpvorrichtung (8) eine peristaltische Pumpe (10) mit einem einzigen Kopf (11) umfasst, der mit variabler Geschwindigkeit drehbar ist.

4. Mischmaschine gemäß Anspruch 3, **dadurch gekennzeichnet, dass** die Bestimmungsvorrichtung des gepumpten Lösungsvolumens ein Bestimmungsmittel der Anzahl der vom Kopf (11) durchgeführten Umdrehungen und ein Berechnungsmittel des der genannten Umdrehungszahl entsprechenden Pumpvolumens umfasst.

5. Mischmaschine gemäß Anspruch 3 oder 4, **dadurch gekennzeichnet, dass** sie eine Platzierungsvorrichtung (12) einer Strömungsschleife (13) der Lösung um einen Teil des Kopfs (11) herum umfasst, um so den Umlauf der Lösung durch teilweise Quetschung eines Bereichs der genannten Schleife zu ermöglichen.
